# EUROPÄISCHE PATENTSCHRIFT

(11) **EP 2 582 684 B1**
(45) Veröffentlichungstag und Bekanntmachung des Hinweises auf die Patenterteilung: **02.07.2014**
(21) Anmeldenummer: 11725889.7
(22) Anmeldetag: 31.05.2011
(51) Int. Cl.: C07D 253/08

(54) **NEUE VERBINDUNGEN ALS LIGANDEN FÜR ÜBERGANGSMETALLKOMPLEXE UND DARAUS HERGESTELLTE MATERIALIEN, SOWIE VERWENDUNG DAZU**
NEW COMPOUNDS AS LIGANDS FOR TRANSITION METAL COMPLEXES AND MATERIALS PRODUCED THEREOF AS WELL AS USES THEREOF
NOUVEAUX COMPOSÉS EN TANT QUE LIGANDS POUR COMPLEXES MÉTALLIQUES À TRANSITION ET MATIÈRES PRÉPARÉES À PARTIR DE CEUX-CI ET LEURS UTILISATIONS

(30) Priorität: 16.06.2010 DE 102010023959
(43) Veröffentlichungstag der Anmeldung: 24.04.2013
(73) Patentinhaber: OSRAM GmbH, 80807 München (DE)
(72) Erfinder: KANITZ, Andreas, 91315 Höchstadt (DE); STARK, Daniel, 96275 Marktzeuln (DE)
(74) Vertreter: Epping - Hermann - Fischer
(86) Internationale Anmeldenummer: PCT/EP2011/058903
(87) Internationale Veröffentlichungsnummer: WO 2011/157546

(56) Entgegenhaltungen:
- WO-A1-2010/007107
- MOHAMED KHODJA, SAAD MOULAY, HOCINE BOUTOUMI AND HORST WILDE: "Two-step syntheses of 3-methyl and 3-phenyl-1,2,4-benzotriazines", HETEROATOM CHEMISTRY, Bd. 17, Nr. 2, 16. März 2006 (2006-03-16), Seiten 166-172, XP002654823, DOI: 10.1002/hc.20200

## Beschreibung

Die Erfindung betrifft neue Verbindungen als Liganden für Übergangsmetallkomplexe und daraus hergestellte Materialien, die beispielsweise als Emitter in organischen lichtemittierenden elektrochemischen Zellen (OLEECs) eingesetzt werden können.

OLEECs sind in den letzten Jahren entwickelt worden und haben das Potential die Leistung der organischen elektro-lumineszierenden Dioden (OLEDs) zu erreichen und noch kostengünstiger als diese produzierbar zu sein.

Aus der WO 2010/007107, DE 10 2009 031 683 A1 und der DE 10 2010 005 632 A1 sind bereits Übergangsmetallkomplexe als emittierende Materialien zu diesen Bauelementen bekannt geworden.

Die Druckschrift D2 (Khodja, M. et al., Heteroatom Chemistry, Vol. 17, No. 2, pp. 166-172, 2006) beschreibt die Synthese von 3-Phenyl-1,2,4-Benzotriazin.

Besonderes Problem stellen noch stabile kurzwellig, also blau, emittierende OLEEC-Emitter dar. Solche blauen OLEEC-Emitter konnten bisher nur auf Basis von fluorierten Liganden erhalten werden, deren aromatische Grundstruktur durch die Fluorsubstitution besonders elektronenarm ist. Die Stabilität solcher Materialien im Bauteil ist jedoch nur sehr gering. Die Emissionswellenlänge verschiebt sich in kurzer Zeit in einen längerwelligen Spektralbereich, was einen Austausch des Fluors im elektrischen Feld vermuten lässt.

Dagegen sind OLEEC-Emitter ohne fluorierte Ligandsysteme sehr langlebig, jedoch nicht blau emittierend.

Aufgabe der Erfindung sind neue Ligandsysteme, die ohne Fluorierung eine ähnlich geringe Elektronendichte in der aromatischen Grundstruktur aufweisen und daher zu dem gleichen Effekt einer kurzwelligen, blauen Lumineszenz führen.

Eine Substanzklasse der elektroarmen Heterocyclen der 3-Aryl-benzo-1,2,4-triazin-Reihe A ist nachfolgend gezeigt, 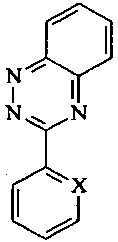 wobei
X für -CH= und -N= steht und
die o-Stellungen der benachbarten Phenylringe durch eine chemische Bindung miteinander verbunden sein können.

Gegenstand der Erfindung eine neuartige Klasse von kationischen Übergangskomplexen mit einem Liganden A, wie beispielsweise eine Kombination des Liganden A mit 2-Phenylimidazol-Ligandstrukturen **B** oder mit sich selbst 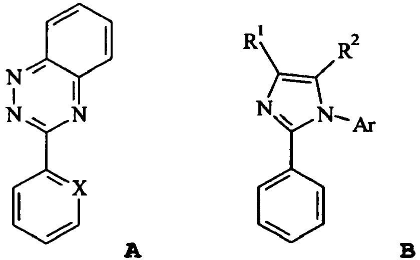 wobei in der Struktur **A**
X für -CH= und -N= steht, und in der Struktur **B**
**Ar** für "Phenyl" oder "2,6-Diisopropyl-1-phenyl" steht, die o-Positionen der benachbarten Phenylringe durch eine chemische Bindung miteinander verbunden sein können,
**R1** und **R2** unabhängig voneinander "H" oder "Phenyl" sein können oder gemeinsam einen annellierten Benzenring bilden.

Schließlich ist Gegenstand der Erfindung die Verwendung eines Übergangsmetallkomplexes mit einem Liganden A oder die Verwendung einer Kombination eines Liganden A mit einem Liganden B in einer organischen lichtemittierenden elektrochemischen Zelle.

Im folgenden Reaktionsschema ist eine bevorzugte Darstellung der neuartigen OLEEC-Emitter durch die Nonoyama-Synthese-Route abgebildet.

### 1.) Reaktionsschema Komplexbildung:

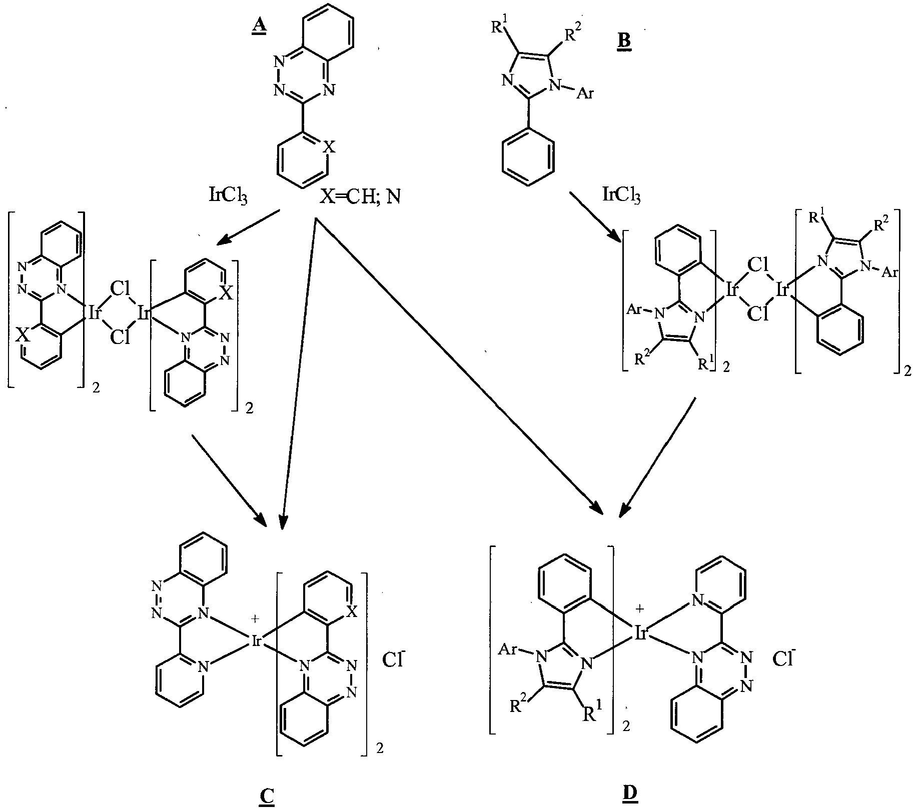

Seit einigen Jahren ist der Zugang kationischer Iridiumkomplexe durch die Nonoyama Route literaturbekannt.

Bisher unbekannt sind alle Strukturen, die durch die Einbeziehung des Liganden **A** aufgebaut werden können.

Diese erfindungsgemäßen Strukturen **C** und **D** sind blau emittierende Materialien, die zur Anwendung in einem OLEEC-Device durch Umsalzung mit einem geeigneten Anion versehen werden müssen. Tetrafluoroborat, Hexafluorophosphat und Hexafluoroantimonat sind besonders geeignete Anionen. Ebenso sind Anionen von ionischen Flüssigkeiten geeignet, deren Organokomponente dem Anion entspricht.
zur Bildung des Übergangsmetallkomplexes wird beispielsweise Iridium eingesetzt, allerdings können auch andere Übergangsmetallatome wie Ruthenium, Rhodium, Palladium, Rhenium, Osmium, Platin, Gold sowie die Lanthaniden als Zentralatom gemäß der Erfindung eingesetzt werden.

Die Darstellung des Liganden **A** geschieht nach dem Reaktionsschema 2:

2-Nitrophenylhydrazin wird in ein Amidrazon durch Reaktion mit Imidoestern umgesetzt, diese werden am Platinkontakt hydriert und vom Katalysator abfiltriert. Das vom Lösemittel befreite Reduktionsprodukt wird mit silylierter Polyphosphorsäure gerührt.

### 2.) Reaktionsschema Ligandensystem A

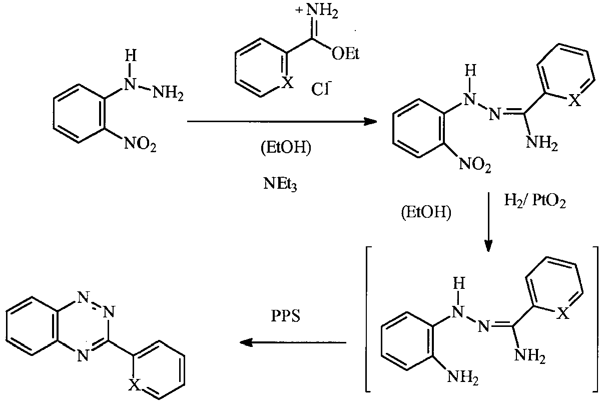

### Ausführungsbeispiele:

### Darstellung von N'-(2-Nitrophenyl)-picolinhydrazonamid

16 g (0,104 mol; 1 Äquivalent) 2-Nitrophenylhydrazin und 15,55 g (0,104
mol; 1 Äquivalent) Picolinimidsäureethylester) werden in 200 ml THF vorgelegt. Man
erhitzt dann für sechs Stunden auf 50 °C. Hierbei fällt nach erfolgter Reaktion ein dunkler Niederschlag aus. Eine dünnschichtchromatographische Kontrolle (Adsorbens: Kieselgel 60; Eluent: Toluol/Essigsäureethylester 4:1) zeigt nahezu vollständigen Umsatz an. Das Lösungsmittel wird am Rotationsverdampfer entfernt, der verbleibende
Niederschlag aus wenig Ethanol umkristallisiert. Man saugt ab und wäscht mit wenig kaltem Ethanol.
Ausbeute: 24,2 g (90 %); dunkelgrünes, glitzerndes Pulver
M = 257,25 g/mol (C12H11N502)
Schmelzpunkt: 197 °C
**MS** (ESI): m/z = 258,3 ([M+H]⁺)

### Darstellung von N'-(2-Nitrophenyl)-benzohydrazonamid

10 g (0,065 mol; 1 Äquivalent) 2-Nitrophenylhydrazin und 12, 07 g (0, 065 mol; 1
Äquivalent) Benzoeimidsäureethylester-Hydrochlorid werden in 200 ml einer Mischung
aus wasserfreiem Ethanol und THF im Verhältnis 1:1 vorgelegt und mit 6,58 g (0,065
mol; 1 Äquivalent) Triethylamin versetzt. Man kocht zwei Stunden am Rückfluss. Nach dem Erkalten der Reaktionsmischung fällt ein dunkelroter Niederschlag aus, der abgesaugt und mit kaltem Ethanol gewaschen wird. Der Schmelzpunkt wird zu 180-182 °C bestimmt.
Ausbeute: 6,8 g (41 %); dunkelrotes, amorphes Pulver
M = 256,26 g/mol (C13H12N402)
**MS** (ESI): m/z = 257,3 ([M+H]⁺)

### Darstellung von 3-(Pyridin-2-yl)benzo[e][1,2,4]-triazin durch Reduktion von N'-(2-Nitrophenyl)-picolinhydrazonamid mittels Wasserstoff am Platin-Katalysator

Die Reduktion des Amidrazons wird in einer Hydrierungsapparatur durchgeführt:

8 g (0,0311 mol) N'-(2-Nitrophenyl)-picolinohydrazonamid werden in einem 1000-ml-Erlenmeyerkolben in 500 ml Ethanol vorgelegt. Zu dieser Suspension wird eine katalytische Menge Platin(IV)-oxid gegeben. Nachdem der Erlenmeyerkolben in die Hydrierungsapparatur eingebaut wurde, wird das benötigte Volumen Wasserstoff in die Apparatur geleitet und die Reduktion des vorliegenden Amidrazons unter kräftigem Rühren durchgeführt. Nach beendeter Reduktion wird das elementare Platin abfiltriert und das Filtrat weitestgehend eingeengt.

Man wiegt die entstandene ölige Masse aus, versetzt mit 60 ml einer Lösung von Trimethylsilylpolyphosphat (Herstellung wie nachfolgend beschrieben) und kocht für eine Stunde am Rückfluss. Das Reaktionsgut wird danach erneut so weit wie möglich eingeengt und mit wenigen Millilitern Wasser versetzt. Der daraufhin langsam auskristallisierende hellgelbe Feststoff wird aus Ethanol umkristallisiert und anschließend abgesaugt; dabei bleibt ein weißer Niederschlag von zurück. Ausbeute: 1,61 g (25 %); weißer, feinpulvriger Niederschlag
M = 208,22 g/mol (C12H8N4)
Schmelzpunkt: 145 °C
**MS** (EI): m/z = 208 ([M]⁺)

### Darstellung von Trimethylsilylpolyphosphat:

In einem Kolben mit Rückflusskühler und Trockenrohr werden 11,37 g (0,070 mol; 1 Äquivalent) Hexamethyldisiloxan und 10 g (0,070 mol; 1 Äquivalent) Phosphorpentoxid, P205, in 100 ml 1,2-Dichlorethan für etwa zwei Stunden am Rückfluss erwärmt, bis alles P205 aufgelöst ist.

### Darstellung eines Di-µ-chloro-Komplexes von B

In einem 500-ml-Einhalskolben werden unter inerten Bedingungen 0,69 g (2,3 mmol; 1
Äquivalent) Iridium(III)-chlorid-Hydrat, IrCl3*nH2O, und 0,40 g (6,9 mmol; 3 Äquivalente)
Natriumchlorid, NaCl, in 20 ml entgastem Wasser vorgelegt und 1,76 g (4,6 mmol; 2 Äquivalente) 1-(2,6-diisopropylphenyl)-2-phenyl-1H-imidazol, das in einer Mischung aus 12 ml entgastem Wasser und 28 ml 2-Ethoxyethanol gelöst worden ist, zugegeben. Es wird auf etwa 50°C erhitzt und nach drei Stunden durch DC-Kontrolle (Adsorbens: Kieselgel 60; Elutionsmittel: Toluol/Aceton 9:1) weitgehender Umsatz zu dem Komplex verzeichnet. Man gibt 200 ml Diethylether und 20 ml Methanol zu, saugt den entstandenen gelb-grün gefärbten Feststoff ab und wäscht diesen mit Methanol. Die Verbindung kann mit einer Ausbeute von 32 % erhalten werden und zersetzt sich oberhalb von 310°C.
Ausbeute: 0,54 g (32 %); heller gelb-grüner Niederschlag
M = 1669,02 g/mol (C84H92Cl2Ir2N8)
Schmelzpunkt: > 310 °C (Zersetzung)
**MS** (ESI): m/z = 799,4 ([(M/2)-Cl]⁺)

### Darstellung der Iridium-Koordinationsverbindung aus A (X=N) und B (Di-µ-chloro-Komplex

0,50 g (0,30 mmol; 1 Äquivalent) **B** (Di-µ-chloro-Komplex) werden in 30 ml Chloroform vorgelegt. Zur entstandenen Suspension gibt man 0,12 g (0,60 mmol; 2 Äquivalente) 3-(pyridin-2-yl)-benzo[e][1,2,4]triazin **A (X=N).** Man kocht etwa 30 Minuten am Rückfluss. Der ausgefallene, leicht gelb gefärbte Feststoff wird abgesaugt und mit Diethylether gewaschen. Die Ausbeute beträgt 90 %. Der Schmelzpunkt liegt bei 194 °C, dabei zersetzt sich das Material gleichzeitig. Ausbeute: 0,56 g (90 %); gelber Feststoff,
M = 787,29 g/mol (C35H25ClIrN70)
Emissionsmaximum (PL): λₘₐₓ = 484 nm (Acetonitril).

Durch die vorliegende Erfindung werden erstmals nicht fluorierte elektronenarme Emittermaterialien vorgestellt, die in organischen lichtemittierenden elektrochemischen Zellen eingesetzt werden können.

## Patentansprüche

1. Kationischer Übergangskomplex mit einem Liganden A, der in Kombination mit einer 2-Phenylimidazol-Ligandstruktur **B** oder mit sich selbst vorliegt, 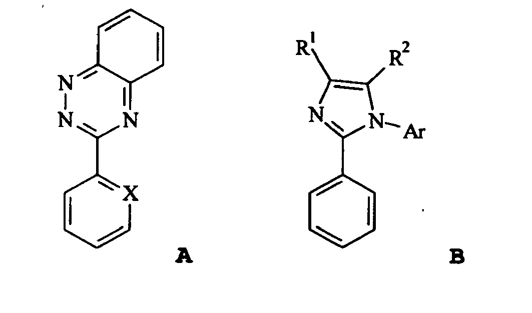 wobei in der Struktur **A**
**X** für -CH= und -N= steht, und in der Struktur **B**
**Ar** für "Phenyl" oder "2,6-Diisopropyl-1-phenyl" steht,
**R1** und **R2** unabhängig voneinander "H" oder "Phenyl" sein können oder gemeinsam einen annellierten Benzenring bilden
und
das Zentralatom ausgewählt ist aus der Gruppe umfassend Iridium, Ruthenium, Rhodium, Palladium, Rhenium, Osmium, Platin, Gold sowie die Lanthaniden.

2. Material für eine lichtemittierende Schicht einer organischen lichtemittierenden elektrochemischen Zelle, einen Übergangsmetallkomplex mit einem Liganden A gemäß dem vorstehenden Anspruch umfassend.

3. Material für eine lichtemittierende Schicht einer organischen lichtemittierenden elektrochemischen Zelle, einen Übergangsmetallkomplex mit einem Liganden A in Kombination mit einem Liganden B gemäß Anspruch 2 umfassend.

4. Verwendung eines Übergangsmetallkomplexes mit einem Liganden A oder die Verwendung einer Kombination eines Liganden A mit einem Liganden B gemäß Anspruch 1 in einer organischen lichtemittierenden elektrochemischen Zelle.

## Claims

1. Cationic transition complex with a ligand A, present in combination with a 2-phenylimidazole ligand structure B or with itself, 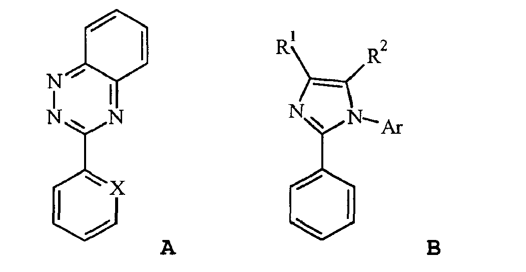 where, in structure **A,**
**X** is -CH= and -N= and, in the structure **B**
**Ar** is "phenyl" or "2,6-diisopropyl-1-phenyl",
**R1** and **R2** may each independently be "H" or "phenyl" or together form a fused benzene ring
and
the central atom is selected from the group comprising iridium, ruthenium, rhodium, palladium, rhenium, osmium, platinum, gold and the lanthanides.

2. Material for a light-emitting layer of an organic light-emitting electrochemical cell, comprising a transition metal complex with a ligand A according to the preceding claim.

3. Material for a light-emitting layer of an organic light-emitting electrochemical cell, comprising a transition metal complex with a ligand A in combination with a ligand B according to Claim 2.

4. Use of a transition metal complex with a ligand A or the use of a combination of a ligand A with a ligand B according to Claim 1 in an organic light-emitting electrochemical cell.

## Revendications

1. Complexe de transition cationique présentant un ligand A, qui se trouve en combinaison avec une structure de ligand 2-phénylimidazole B ou avec lui-même, 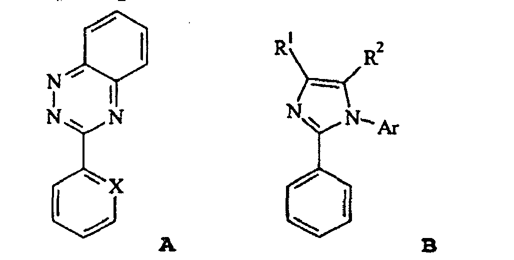 où, dans la structure A,
X représente -CH= et -N=, et dans la structure B
Ar représente "phényle" ou "2,6-diisopropyl-1-phényle",
R1 et R2 peuvent représenter, indépendamment l'un de l'autre, "H" ou "phényle" ou former, ensemble, un cycle benzène condensé et
l'atome central est choisi dans le groupe comprenant l'iridium, le ruthénium, le rhodium, le palladium, le rhénium, l'osmium, le platine, l'or ainsi que les lanthanides.

2. Matériau pour une couche émettrice de lumière d'une cellule électrochimique organique émettrice de lumière, comprenant un complexe de métal de transition présentant un ligand A selon la revendication précédente.

3. Matériau pour une couche émettrice de lumière d'une cellule électrochimique organique émettrice de lumière, comprenant un complexe de métal de transition présentant un ligand A en combinaison avec un ligand B selon la revendication 2.

4. Utilisation d'un complexe de métal de transition présentant un ligand A ou utilisation d'une combinaison d'un ligand A avec un ligand B selon la revendication 1 dans une cellule électrochimique organique émettrice de lumière.
